# EUROPEAN PATENT SPECIFICATION

(11) **EP 0 689 854 B1**
(45) Date of publication and mention of the grant of the patent: **31.10.2001**
(21) Application number: 95850121.5
(22) Date of filing: 27.06.1995
(51) Int. Cl.: A61N 1/08, A61N 1/372, G01R 31/04, H01R 13/703

(54) **Implant with indicating device**
Implantat mit Anzeigevorrichtung
Implants avec un dispositif d'indication

(30) Priority: 29.06.1994 SE 9402297
(43) Date of publication of application: 03.01.1996
(73) Proprietor: St. Jude Medical AB, 175 84 Järfälla (SE)
(72) Inventor: Fröberg, Paul, S-161 70 Bromma (SE); Franberg, Per, S-118 67 Stockholm (SE); Högnelid, Kurt, S-137 38 Västerhaninge (SE); Killander, Fredrik, S-183 75 Täby (SE); Magnusson, Peter, S-131 48 Nacka (SE); Nyman, Per, S-182 64 Djursholm (SE)

(56) References cited:
- EP-A- 0 303 235
- EP-A- 0 630 662
- US-A- 4 245 643

## Description

The invention relates to a Medical implant for emitting electrical pulses, said implant comprising a device for indicating that an electrode cable is correctly connected to the implant for emitting electrical pulses, the implant thereby being equipped with a connection part for the electrode cable's proximal end.

The connecting parts of pacemakers often consist of a molded-on part with electrode connectors. The molded-on part is made of transparent epoxy plastic and attached to the top of the pacemaker enclosure. The electrode connectors in the molded-on part are electrically connected to the pacemaker circuits inside the enclosure via a number of connecting pins. The proximal end of the electrode cable is attached to the molded-on part with e.g. screws. The American patent document US 5 086 773 cites such solutions in a description of the prior art. The device described in US 5 086 773 is an example of electrode cable attachment without screws or the need for tools. The described attachment device contains at least one coil spring, arranged in a connection hole, for the proximal end of the electrode cable. The internal diameter of the coil spring is slightly smaller than the external diameter of the proximal end of the electrode cable. When the proximal end is rotated part of one turn in the coil spring's coiling direction at the same time as the end is pushed into the connection hole, the spring expands enough to permit insertion of the electrode cable's proximal end. When pressure on the connection part is relaxed, the spring resumes its normal state, thereby affixing the electrode cable and establishing electrical contact.

EP-0 448 760 also provides an example of a fixation device in which a spring located in a connection hole is employed for attaching and seating an electrical conductor in the connection hole.

With electrode connection in a transparent molded-on part, an external visual check can be made to ensure that the connecion is correct. However, one disadvantage with the use of a molded part of the described kind is that the part takes a relatively long time to make, since the epoxy plastic needs time to cure. Another disadvantage is that cracks could develop in the molded-on part over time. These disadvantages can be avoided by integrating the connector into the pacemaker enclosure which would thus have a hole for the proximal end of the electrode cable. This type of connection is usually referred to as a "black hole."

The U.S. patent document 4 934 366 describes a pacemaker connection with a black hole-type electrode connection and shows how the proximal end of the electrode cable is attached to the pacemaker and seals the attachment area without the need for screws. Here, fixation is achieved with the aid of clamping rings, threaded onto the proximal end of the electrode cable when the cable is connected to the pacemaker, and with the aid of springs surrounding the proximal end, the springs then also serving as electrical connectors for the electrode cable.

One problem with black hole connections according to the description in US 4 934 366 is that no indication is provided as to whether the electrode cable has been correctly attached and seated. Physicians implanting implants have expressed a wish for some kind of indication showing that connection is correct.

One device, which emits a clicking sound to confirm that the electrode cable's proximal end has been correctly connected to a device for emitting electrical pulses, is described in our own patent application, EP-0 630 662 (published after the priority date of the present application). The mechanically generated clicking noise is produced by a dish-shaped disk which snaps from one position to another when the end of the electrode presses against the disk. EP-A-0 303 235 discloses a device for indicating that a cable is correctly connected to the connector housing containing a contact means for electrically seating the proximal end of the cable and an indication means, the contact means being arranged to electrically cause the indication means to emit an indication signal, perceptible outside the connector housing, when the cable is correctly connected.

The object of the present invention is to set forth an improved indication device.

This object is achieved with an indication device of kind cited above and with the features set forth in the first claim.

Thus, the invention provides an indication when the electrode cable has been correctly connected to a medical implant for emitting electrical pulses.

The indication means generates electrical pulses which, via a contact means, are sent to the electrode cable, so pulses emitted from the distal end of the electrode cable can be detected by ECG equipment.

The invention will now be described in greater detail, referring to the FIGURES in the attached drawings in which:
FIG. 1 is a schematic rendition of a medical implant with the invention implemented;
FIG. 2 is a schematic rendition of the connection part and the electrode cable according to the invention;

Identical or similar elements bear the same reference designations in the FIGURES.

The invention will now be described referring to a medical implant for connection of only one electrode cable. The invention is naturally also applicable to medical implants accepting two or more connected electrode cables.

The medical implant could be e.g. a pacemaker, a defibrillator or a nerve stimulator.

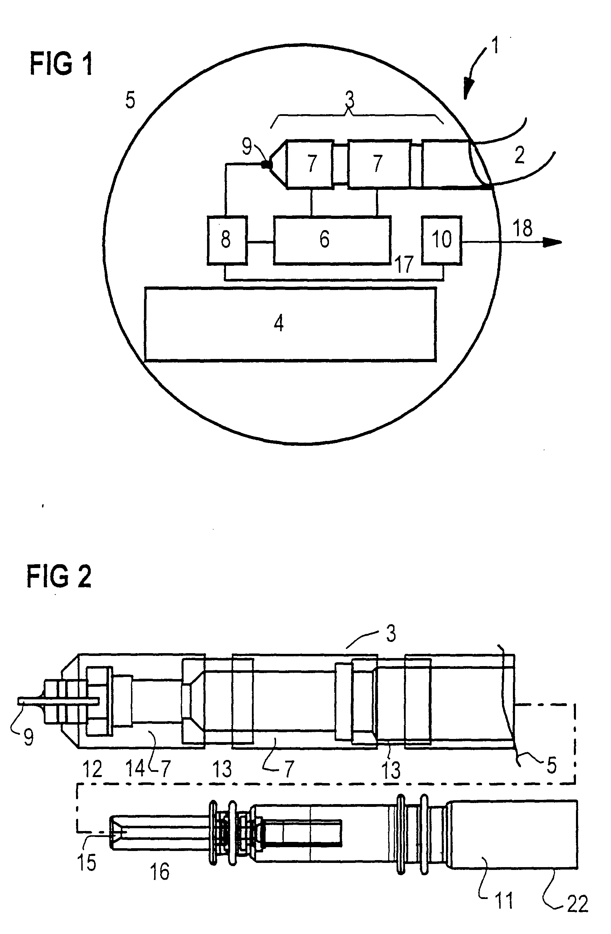

FIG. 1 shows a schematic rendition of a medical implant 1 containing an electrode cable 2, a connection part 3 and a battery 4 for powering the implant 1. The electrode cable 2 is shown connected to the connection part 3. The implant 1 further has an enclosure 5 and a control device 6, for controlling operation of the implant 1, connected to one or a plurality of connection means 7 in the connection part 3. The control device 6 is also connected to an indication circuit 8 and to a contact means 9, located in the connection part 3. The indication circuit 8, the contact means 9 and an indication means 10 jointly constitute the indication device according to the invention. The indication means 10 is connected to the indication circuit 8.

The description of the invention below refers to both FIG. 1 and FIG. 2.

FIG. 2 shows a more detailed schematic rendition of the connection part 3 and the proximal end 11 of the electrode cable 2. The connection part 3 has a fixation means 12 for attaching the proximal end 11 of the electrode cable and one or a plurality of insulated segments 13 which can be made of a transparent ceram. The fixation means 12 consists of a coiled spring concentrically arranged in the connection part 3 inside the connection means 7. The internal diameter of the spring is only slightly smaller than the diameter of the outermost part 16 of the proximal end 11 of the electrode cable. "Outermost part of the proximal end of the electrode cable" refers to the deepest part inside the connection part 3 when connection is correct. The electrode cable 2 is attached to the implant 1 when the proximal end is rotated part of one turn in the coil spring's direction of coiling at the same time as the end is pushed into the connection part 3. This rotation causes the spring to expand enough to permit insertion of the electrode cable's proximal end 11. When rotation ceases and pressure on the electrode cable 2 is relaxed, the spring strives to resume its normal state, thereby attaching the proximal end 11 of the electrode cable in the connection part 3.

The contact means 9 inside the connection part 3 is equipped with a pin 14, concentrically arranged inside the connection part 3, arranged in such a way that the pin 14 is inserted into an opening 15 in the outermost part 16 of the proximal end 11 of the electrode cable when the electrode cable 2 is attached, electrical contact thereby being established between the contact means's 9 pin 14, the outermost part 16 of the proximal end 11 of the electrode cable, the fixation means 12 and connection means 7. When the electrode cable 2 has been affixed by the fixation means 12 and electrically seated with the connection means 7, the contact means 9 with its pin 14 and the indication circuit 8 as well are in contact with a potential applied by the control device 6 to the connection means 7. This is detected by the indication circuit 8 which generates a control signal 17 applied to the indication means 10 in which an indication signal 18, perceptible outside the medical implant 1, is generated.

According to the invention, the indication means 10 generates electrical pulses which, via the contact means 9, are applied to the electrode cable 2 and carried to the electrode cable's 2 distal end located e.g. inside the heart of a patient. The pulses sent to the electrode cable 2 via the contact means 9 can be detected with the ECG equipment which is always available for monitoring the patient's ECG during an implantation. According to one alternative, two closely spaced consecutive pulses are sent to the contact means 9 and carried to the distal end of the electrode cable 2, thereby making it easy to identify the pulses with ECG equipment and supply an indication that connection is correct. According to another alternative, a pulse is sent to the contact means 9. The control device 6 then sends a pulse shortly thereafter to one of the connection means 7. Identification of the pulses with ECG equipment supplies an indication showing that the connection is correct. After a defined period of time, e.g. 30 seconds, after the electrode cable 2 has been connected, the indication circuit 8 stops generating pulses.

## Claims

1. Medical implant for emitting electrical pulses having an enclosure (5), said implant comprising a device for indicating that an electrode cable (2) is correctly connected to an implant (1) for emitting electrical pulses, the implant thereby being equipped with a connection part (3) for the electrode cable's proximal end (11), the indication device containing a contact means (9) for electrically seating the proximal end (11) of the electrode cable and an indication means (10), the contact means (9) thereby being arranged to electrically cause the indication means (10) to emit an indication signal (18), which is perceptible outside the implant (1), when the electrode cable (2) is correctly connected to the implant (1), said indication means (10) generating electrical pulses which, via the contact means (9), are sent to the electrode cable (2), the pulses emitted from the distal end of the electrode cable (2) then being detectable with ECG equipment.

2. An indication device according to claim 1, **characterized in that** the contact means (9) is in electrical contact with the proximal end (11) of the electrode cable only if the electrode cable (2) is correctly connected to the connection part (3), the connection part (3) containing a fixation means (12) for affixing the proximal end (11) of the electrode cable and one or a plurality of connection means (7) for electrically seating the proximal end (11) of the electrode cable.

3. An indication device according to claims 1 or 2, **characterized in that** the device also comprises an indication circuit (8) connected to the contact means (9) and to the indication means (10), the contact means (9) thereby being arranged to electrically cause the indication circuit (8) to send a control signal (17) to the indication means (10) for generation of an indication signal (18) when the electrode cable (2) has been correctly affixed and electrically seated.

## Patentansprüche

1. Medizinisches Implantat zur Abgabe elektrischer Impulse mit einem Gehäuse (5), wobei dieses Implantat eine Vorrichtung zum Anzeigen aufweist, daß ein Elektrodenkabel (2) korrekt mit einem Implantat (1) zur Abgabe elektrischer Impulse verbunden ist, wozu das Implantat mit einem Verbindungsteil (3) für das proximale Ende (11) des Elektrodenkabels ausgestattet ist, die Anzeigevorrichtung ein Kontaktmittel (9) zum elektrischen Anschließen des proximalen Endes (11) des Elektrodenkabels und ein Anzeigemittel (10) aufweist, wodurch das Kontaktmittel (9) ausgebildet ist, um das Anzeigemittel (10) elektrisch zur Abgabe eines Anzeigesignals (18) zu veranlassen, das außerhalb des Implantats (1) wahrnehmbar ist, wenn das Elektrodenkabel (2) korrekt mit dem Implantat (1) verbunden ist, wobei das Anzeigemittel (10) elektrische Impulse erzeugt, die über das Kontaktmittel (9) zu dem Elektrodenkabel (2) gesendet werden, wodurch die vom distalen Ende des Elektrodenkabels (2) abgegebenen Impulse mit Hilfe einer EKG-Ausrüstung detektierbar sind.

2. Eine Anzeigevorrichtung nach Anspruch 1, **dadurch gekennzeichnet, daß** das Kontaktmittel (9) nur mit dem proximalen Ende (11) des Elektrodenkabels in elektrischem Kontakt steht, wenn das Elektrodenkabel (2) korrekt mit dem Verbindungsteil (3) verbunden ist, wobei das Verbindungsteil (3) ein Befestigungsmittel (12) zum Fixieren des proximalen Endes (11) des Elektrodenkabels und ein oder eine Vielzahl von Verbindungsmittel(n) (7) zum elektrischen Anschließen des proximalen Endes (11) des Elektrodenkabels aufweist.

3. Eine Anzeigevorrichtung nach Anspruch 1 oder 2, **dadurch gekennzeichnet, daß** die Vorrichtung auch eine mit dem Verbindungsmittel (9) und dem Anzeigemittel (10) verbundene Anzeigeschaltung (8) aufweist, wodurch das Verbindungsmittel (9) ausgebildet ist, die Anzeigeschaltung (8) elektrisch zu veranlassen, ein Steuersignal (17) an das Anzeigemittel (10) zum Erzeugen eines Anzeigesignals (18) zu senden, wenn das Elektrodenkabel (2) korrekt fixiert und elektrisch angeschlossen ist.

## Revendications

1. Implant médical pour émettre des impulsions électriques ayant une enveloppe (5), l'implant comportant un dispositif destiné à indiquer qu'un câble (2) d'électrode est connecté correctement à un implant (1) pour émettre des impulsions électriques, l'implant étant ainsi muni d'une partie (3) de connexion pour l'extrémité (11) proximale du câble d'électrode, le dispositif d'indication contenant des moyens (9) de contact destinés à faire reposer en siège électriquement l'extrémité (11) proximale du câble d'électrode et des moyens (10) d'indication, les moyens (9) de contact étant ainsi agencés pour faire en sorte électriquement que les moyens (10) d'indication émettent un signal (18) d'indication, qui est perceptible à l'extérieur de l'implant (1), lorsque le câble (2) d'électrode est connecté correctement à l'implant (1), les moyens (10) d'indication produisant des impulsions électriques qui, par l'intermédiaire des moyens (9) de contact, sont envoyées au câble (2) d'électrode, les impulsions émises à partir de l'extrémité distale du câble (2) d'électrode étant alors détectable par un équipement ECG.

2. Dispositif d'indication suivant la revendication 1, **caractérisé en ce que** les moyens (9) de contact sont en contact électrique avec l'extrémité (11) proximale du câble d'électrode uniquement si le câble (2) d'électrode est connecté correctement à la partie (3) de connexion, la partie (3) de connexion contenant des moyens (12) de fixation pour fixer l'extrémité (11) proximale du câble d'électrode et l'un ou une pluralité des moyens (7) de connexion pour faire reposer en siège électriquement l'extrémité (11) proximale du câble d'électrode.

3. Dispositif d'indication suivant la revendication 1 ou 2, **caractérisé en ce que** le dispositif comporte également un circuit (8) d'indication connecté aux moyens (9) de contact et aux moyens (10) d'indication, les moyens (9) de contact étant ainsi agencés pour faire en sorte électriquement que le circuit (8) d'indication envoie un signal (17) de commande aux moyens (10) d'indication pour la production d'un signal (18) d'indication lorsque le câble (2) d'électrode a été fixé correctement et repose en siège électriquement correctement.
